# EUROPEAN PATENT APPLICATION

(11) **EP 0 905 206 A2**
(43) Date of publication of application: **31.03.1999**
(21) Application number: 98402238.4
(22) Date of filing: 10.09.1998
(51) Int. Cl.: C09C 1/00, C09C 3/10, C08K 9/10, A61K 7/031, A61K 7/035

(54) **Composite powder and make-up cosmetic composition containing the same**

(30) Priority: 17.09.1997 JP 270451/97
(71) Applicant: SHISEIDO COMPANY, LTD., Chuo-ku Tokyo (JP)
(72) Inventor: Ogawa, Katsuki, Kohoku-ku, Yokohama-shi, Kanagawa (JP); Ikeda, Tomoko, Kohoku-ku, Yokohama-shi, Kanagawa (JP); Murui, Yukio, Kohoku-ku, Yokohama-shi, Kanagawa (JP)
(74) Representative: Rinuy, Santarelli

(57) **Abstract**

A composite powder comprising (a) a flake powder having (b) hollow spherical resin powder coated on the substantially entire surface thereof, wherein the weight ratio of the powder (a): the powder (b) is 80:20 to 20:80, and a make-up cosmetic composition containing the same.

## Description

### BACKGROUND OF THE INVENTION

### 1. Field of the Invention

The present invention relates to a composite powder. More specifically, it relates to a composite powder capable of providing natural finish having gloss and transparent feelings close to natural skin, while covering defects on the skin, when formulated into a make-up cosmetic composition.

### 2. Description of the Prior Art

As is known in the art, conventional make-up cosmetic compositions mainly contain, as a means for covering defects on the skin, titanium dioxide having a refractive index of 2.5 - 2.7 and an average particle diameter of about 0.2 µm. However, although the defects on the skin are able to be covered when this type of make-up cosmetic composition is applied to the skin, the cosmetic finish of the skin having unnatural feelings is formed as the final finish due to the large difference of the refractive index from that of skin (i.e., 1.57). Therefore, it should be taken in the past that the amount of the titanium dioxide formulated into the cosmetic composition is decreased to obtain the finish close to the natural skin and the natural finish having a transparent feeling.

However, although the cosmetic finish of the skin is improved to some extent in the direction of the natural finish, when the amount of titanium dioxide is decreased, the uniform finish is difficult to obtain due to the strong cohesive force of the titanium dioxide and, according to the above-mentioned approach, the covering power is undesirably decreased to cause a problem that defects on the skin become noticeable. Thus, the covering of defects on the skin and the formation of natural cosmetic finish on the skin are so-called antinomy phenomenon.

Recently, in order to obtain the cosmetic finish close to the actual natural skin, the development of powder has been made with paying attention to the tissue structures of the skin. However, there is a limit to imitate the complicated tissue structures of the skin. Accordingly, when the make-up cosmetic compositions containing the developed powder formulated thereinto are actually applied to the skin, the cosmetic finish obtained therefrom is substantially the same as those obtained from the application of conventional cosmetic compositions.

Accordingly, there are strong needs for the developments of powder components capable of providing the natural cosmetic finish close to the natural skin, while covering defects on the skin.

### SUMMARY OF THE INVENTION

Accordingly, the objects of the present invention are to eliminate the above-mentioned problems in the prior art and to provide a powder component capable of providing natural cosmetic finish close to the natural skin, while covering defects on the skin, when applied to the skin.

Another object of the present invention is to provide a make-up cosmetic composition capable of providing natural cosmetic finish close to the natural skin, while covering defects on the skin, when applied to the skin.

In accordance with the present invention, there is provided a composite powder comprising (a) a flake powder having (b) hollow spherical resin powder coated on the substantially entire surface thereof, wherein the weight ratio of the powder (a): the powder (b) is 80:20 to 20:80.

In accordance with the present invention, there is also provided a make-up cosmetic composition comprising a make-up base component and the composite powder mentioned above.

### BRIEF DESCRIPTION OF THE DRAWINGS

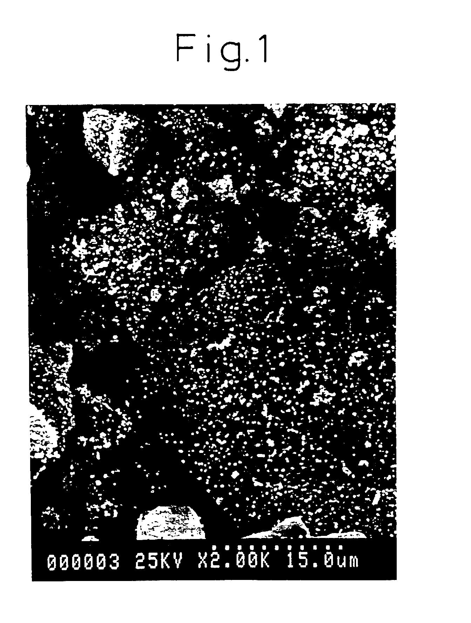

The present invention will now be better understood from the description set forth below with reference to Figure 1, which is a scanning type electron microscope photograph (x 5000) showing the particulate structure of the composite powder (i.e., mica coated with a hollow spherical styrene-acryl copolymer resin) obtained in Preparation Example 1.

### DESCRIPTION OF THE PREFERRED EMBODIMENTS

As a result of the intensive study, the present inventors found that the composite powder obtained by coating the surface of a flake powder with a specified amount of one or more finely divided particles of hollow spherical resin powder can provide natural cosmetic finish to the skin, while covering defects on the skin when formulated into a make-up cosmetic composition as a powder component.

Various attempts have been made in the art to cover pigments with resins, for example, to mechanically reinforce pearl pigments (see JP-A-52-93440), to control the gloss of extender pigments selected from talc, kaolin, mica, and sericite (JP-A-5-76444), to control the gloss of pearl pigments comprising titanated mica, iron oxide coated mica (JP-A-9-48707), etc.

However, the composite powder according to the present invention is obtained by coating a flake powder with a hollow spherical resin powder, with paying attention to the excellent optical characteristics of the hollow spherical resin powder. The composite powders (i.e., coated pigments) according to the present invention thus obtained are those having the strong light scattering capability possessed by the hollow spherical resin powder and the characteristics possessed by the flake powder, in combination. These composite powders are not known and first found by the present inventors.

Although the coated pigments obtained by coating the extender pigments such as talc, kaolin, mica, sericite, etc., in the form of a flake powder can provide natural cosmetic finish of skin, the light scattering capability and the light absorbing capability are poor and, as a result, the covering of defects on the skin is difficult. However, according to the present invention, since the coating spherical resin powder has a hollow structure, the light scattering capability and the light absorbing capability thereof are high when compared with conventional spherical resin powder, and therefore, the covering power for defects on the skin is not sufficient. Nevertheless, when the above-mentioned hollow spherical resin powder is formulated alone into the cosmetic composition, the synergy or synergistic effect with the flake powder cannot be obtained and, in addition, the adhesion to the skin is poor. Thus, the above-mentioned desired effects are not realized. Therefore, according to the present invention, the flake powder, as a substrate, is coated with the hollow spherical resin powder to increase the adhesion thereof to the skin and, further, by the synergistic effect with the optical characteristics of the substrate (i.e., the flake powder), to provide the natural cosmetic finish of the skin, while covering defects on the skin. Furthermore, the composite powder of the present invention provides the excellent application properties, i.e., smooth extension on the skin from the viewpoints of the application feelings.

The constitution of the present invention will now be described in detail.

The flake powders usable in the composite powder of the present invention are those selected from the group consisting of, for example, talc, mica, sericite, barium sulfate, alumina, boron nitride, synthetic mica, synthetic talc, zinc oxide, silica, titanated mica, lauroyl lysin and iron oxide coated mica. These may be used alone or in any mixture thereof.

These flake powders usable in the present invention are those preferably having an average particle diameter of 1 to 80 µm, more preferably 10 to 50 µm. When the average particle diameter is more than 80 µm, there are sometimes feelings of, for example, roughness of the powder and dotted feeling (too glossy) especially in the case of titanated mica. Contrary to this, when the average particle diameter is less than 1 µm, the coating of the resin powder on the surface of the substrate tend to become incomplete, it is not preferable that the desired optical characteristics are difficult to be obtained.

The flake powder according to the present invention preferably has an aspect ratio (i.e., a ratio of an average thickness/an average diameter) of 10 to 300, more preferably 30 to 170.

According to the present invention, as the flake powder, the use of titanated mica in combination with the other flake powder is preferable. When these two or more hollow spherical resin coated powder are formulated together, especially, the reddish interferenced light of titanated mica provides the compensation effect to the dullness of the skin to thereby form a foundation giving the beautiful, physically healthy looking and highly transparent finish to the skin.

The hollow spherical resin powders for coating the flake powders according to the present invention include, for example, those composed of an acryl resin, a nylon resin, a styrene resin, a silicone resin or any copolymer thereof. Among these, the use of acryl resins is preferable and the use of hollow spherical fine particles of styrene-acryl copolymer resins is especially preferable. The shape of the hollow spherical resin powder should be spherical so as to uniformly and systematically deposit on the surface of the flake powder as a substrate. Furthermore, from the viewpoint of the optical characteristics, the spherical fine particle particle exhibits the most highly irregular reflection of the light.

The hollow spherical resin powder according to the present invention preferably has an average hollow diameter (i.e., the diameter of the hollow portion) of 0.1 to 0.5 µm, more preferably 0.2 to 0.4 µm and an average particle diameter (i.e., the outer diameter of the particle) of 0.3 to 1.5 µm, more preferably 0.3 to 0.7 µm. When the average hollow diameter is more than 0.5 µm, the light scattering capability of the visible light tends to decrease, whereas, when less than 0.1 µm, the scattering capability of the visible light tends to decrease although the scattering capability of UV light is increased. When the average particle diameter is more than 1.5 µm, the adhesion thereof to the flake powder tends to be decreased and, therefore, the irregular reflection effect is not sufficiently obtained. On the other hand, when the average particle diameter is less than 0.3 µm, the ideal hollow structure capable of scattering the visible light tends to be difficult to accomplish, and therefore, the desired optical characteristics are difficult to obtain.

The molecular weight of the polymer for the above-mentioned hollow spherical resin powder is not specifically limited so long as the polymer is stable in the oil, water, alcohol, various chemical drugs formulated, together with the composite powder, into make-up cosmetic compositions.

The composite powder of the present invention, i.e., the flake powder coated with the hollow spherical resin powder can be produced, for example, by mixing the flake powder and the hollow spherical resin powder at the predetermined weight ratio, followed by slurrying in an aqueous alcohol solution and then by spray-drying the resultant slurry at a temperature of, for example, 70 - 100°C. Thus, the flake powder having the hollow spherical resin powder adhered to the surface thereof can be produced.

The commercially available hollow spherical resin powders usable in the present invention include, for example, OP-84J, OP-62, OP-96, HP-91, HP-1055, etc. available from Rohm & Haas Japan. Note that the composite powders obtained by mixing one or a mixture of two or more flake powders with various hollow spherical resin powders to cover the flake powder with the hollow spherical resin powders are also within the scope of the present invention.

The composition ratio (i.e., weight ratio of (a):(b)) of the flake powder (a) and the hollow spherical resin powder (b) in the composite powder according to the present invention should be 80:20 to 20:80, preferably 60:40 to 40:60. When the proportion of the hollow spherical resin particle is more than this range, the hollow spherical powder is aggregated, whereby the uniform adhesion to the surface of the substrate becomes difficult. Contrary to this, when the proportion of the hollow spherical resin powder is less than the above-mentioned proportion ratio, the uniform coating on the entire surface of the substrate becomes difficult. In either case, the natural cosmetic finish having a transparent feeling, while covering defects on the skin, cannot be obtained.

According to the present invention, there is provided a make-up cosmetic composition containing the above-mentioned composite powder formulated therein together with a make-up cosmetic base component. Although there are no limitations to the amount of the composite powder in the make-up cosmetic composition, the preferable amount is 1.0 to 30.0% by weight, based upon the total amount of the cosmetic composition.

The make-up cosmetic composition according to the present invention may include, for example, W/O type emulsified cosmetic compositions, oily cosmetic compositions, solid powdery cosmetic compositions. When formulating these types of make-up cosmetic compositions, any conventional components may be formulated thereinto. Examples of such components are oil components such as higher alcohols, lanolin derivatives, fatty acid ester oils of polyethylene glycol, silicone oils, paraffin oils, fluorine oils; humectant components such as propylene glycol, glycerol, polyethylene glycol; oil-soluble polymeric substances; water-soluble polymeric substances; ion exchange water; alcohols; extender pigments such as talc, mica, kaolin; iron oxide, spherical powders, antiseptics, bactericids, pH adjusting agent, antioxidants, dyes, flavors, etc. These components may be used in the conventional formulating amounts.

### EXAMPLES

The present invention will now be further illustrated by, but is by no means limited to, the following Examples, wherein "percent" is all on a weight basis unless otherwise specified.

### Preparation Example 1: Preparation of mica coated with hollow spherical styrene-acryl copolymer resin powder

As a substrate pigment, mica in the form of a flake having an average particle diameter of 6 µm and an average thickness of 0.12 µm was used. This substrate and hollow spherical styrene-acryl copolymer resin powder (Trade name: OP-62 available from Rohm & Haas Japan) were slurried in an aqueous ethanol solution, followed by spray drying at a temperature of 70 - 100°C to obtain the mica coated with the hollow spherical styrene-acryl copolymer resin at the coating ratio of the mica/the hollow spherical resin powder = 55/45.

The particle structure of the resultant composite powder, i.e., the mica coated with the hollow spherical styrene-acryl copolymer resin is shown in Fig. 1, which is a photograph, instead of a drawing, taken by a scanning type electron microscope. As is clear from Fig. 1, the mica in the form of a flake was coated with the spherical styrene-acryl copolymer resin to form the composite material. Furthermore, when the optical characteristics of the resultant mica coated with the hollow spherical styrene-acryl copolymer resin was determined by a goniometer, the irregular reflection effect of light is higher than that of conventional mica coated with spherical PMMA (i.e., polymethylmethacrylate) resin powder and the covering effect due to light was observed.

### Preparation Example 2: Preparation of titanated mica coated with hollow spherical styrene-acryl copolymer resin powder

A titanated mica coated with the hollow spherical styrene-acryl copolymer resin powder at a covering ratio of the titanated mica/the hollow spherical styrene-acryl copolymer resin powder = 57/43 was prepared in the same manner as in Preparation Example 1, except that a titanated mica in the form of a flake having an average diameter of 20 µm and an average thickness of 0.38 µm and exhibiting a reddish interference light was used as the substrate pigment.

### Preparation Example 3: Preparation of barium sulfate coated with hollow spherical styrene-acryl copolymer resin powder

A barium sulfate coated with the hollow spherical styrene-acryl copolymer resin powder at a covering ratio of the barium sulfate/the hollow spherical-acryl copolymer resin powder = 50/50 was prepared in the same manner as in Preparation Example 1, except that a barium sulfate in the form of a leaf having an average diameter of 30 µm and an average thickness of 0.5 µm was used as the substrate pigment.

### Preparation Example 4: Preparation of alumina coated with hollow spherical styrene-acryl copolymer resin powder

An alumina coated with the hollow spherical styrene-acryl copolymer resin powder at a covering ratio of the alumina/the hollow spherical-acryl copolymer resin powder = 55/45 was prepared in the same manner as in Preparation Example 1, except that an alumina in the form of a leaf having an average diameter of 26 µm and an average thickness of 0.4 µm was used as the substrate pigment.

### Examples 1 and 2 and Comparative Examples 1- 3

Solid powder foundations were prepared by the following formulations shown in Table 1 using the powders coated with the hollow spherical styrene-acryl copolymer resin obtained in the above Preparation Examples 1 and 2 in a conventional manner. Furthermore, the foundation of Comparative Example 1 in which the hollow spherical styrene-acryl copolymer resin powder alone was formulated, the foundation of Comparative Example 2 in which a conventional mica coated with a spherical styrene-acryl copolymer resin was contained and the foundation of Comparative Example 3 in which the pigment-use titanium dioxide was used for obtaining the covering power were prepared similarly.

**Table 1**

| Ingredient | Example | | Comparative Example | | |
|---|---|---|---|---|---|
| | 1 | 2 | 1 | 2 | 3 |
| Sericite | 33.0 | 33.0 | 33.0 | 33.0 | 33.0 |
| Mica | 22.0 | 22.0 | 26.4 | 22.0 | 22.0 |
| Talc | 15.0 | 15.0 | 15.0 | 15.0 | 15.0 |
| Titanium dioxide for pigment | - | - | - | - | 8.0 |
| Iron oxide | 7.0 | 7.0 | 7.0 | 7.0 | 7.0 |
| Mica coated with hollow spherical styrene-acryl copolymer resin (Preparation Example 1) | 8.0 | 4.0 | - | - | - |
| Titanated mica coated with hollow spherical styrene-acryl copolymer resin (Preparation Example 2) | - | 4.0 | - | - | - |
| Hollow spherical styrene-acryl copolymer resin powder (OP-62) | - | - | 3.6 | - | - |
| Mica coated with spherical styrene-acryl copolymer resin | - | - | - | 8.0 | - |
| Antiseptic | 0.2 | 0.2 | 0.2 | 0.2 | 0.2 |
| Dimethylpolysiloxane (6 cs) | 5.0 | 5.0 | 5.0 | 5.0 | 5.0 |
| Purified lanolin | 4.0 | 4.0 | 4.0 | 4.0 | 4.0 |
| Squalane | 4.0 | 4.0 | 4.0 | 4.0 | 4.0 |
| Sorbitan ostearate | 1.0 | 1.0 | 1.0 | 1.0 | 1.0 |
| Antioxidant | q.s. | q.s. | q.s. | q.s. | q.s. |
| Flavor | q.s. | q.s. | q.s. | q.s. | q.s. |

### Example 3 and Comparative Examples 4 and 5

A make-up cosmetic composition, face powder, was prepared by the following formulation shown in Table 2 using the mica coated with hollow spherical styrene-acryl copolymer resin in a conventional manner.

**Table 2**

| Ingredient | Example | Comparative Example | |
|---|---|---|---|
| | 3 | 4 | 5 |
| Talc | 59.3 | 59.3 | 59.3 |
| Mica | 25.0 | 25.0 | 25.0 |
| Zinc oxide | 5.0 | 5.0 | 5.0 |
| Titanium dioxide for pigment | - | | - 5.0 |
| Mica coated with hollow spherical styrene-acryl copolymer resin (Preparation Example 1) | 5.0 | - | - |
| Mica coated with spherical styrene-acryl copolymer resin | - | 5.0 | - |
| Iron oxide | 0.2 | 0.2 | 0.2 |
| Antiseptic | 0.2 | 0.2 | 0.2 |
| Squalane | 3.0 | 3.0 | 3.0 |
| Purified lanolin | 2.0 | 2.0 | 2.0 |
| Sorbitan sesquiisostearate | 0.2 | 0.2 | 0.2 |
| Antioxidant | q.s. | q.s. | q.s. |
| Flavor | q.s. | q.s. | q.s. |

The cosmetic finishes of the make-up cosmetic compositions obtained in Examples 1 - 3 and Comparative Examples 1 - 5 were compared as follows.

### 1) Determination of Finishing Effect

The solid powder foundations of Example 1 and Comparative Examples 1 - 3 were applied to a test panel consisting of 9 women testees and the optical characteristics of the natural skin and the cosmetic finishes after applications were determined by a goniometer. The angle of incidence of the light was fixed at 45° and the light reflection characteristics of the natural skin and the cosmetic finishes after the applications of the foundations prepared in Example 1 and Comparative Examples 1 - 3 were determined, while changing the acceptance angle from 90° to 160°, and the reflection characteristics were evaluated by the lightness value (Y value). The portion determined was the lower portion of jaw. The evaluation results are shown in Table 3.

As shown in Table 3, the cosmetic finish effect (i.e., texture) of the solid powder foundation exhibited the optical characteristics closer to the natural skin when compared with those of Comparative Examples 1 - 3.

**Table 3**

| Acceptance angle (degree) | 90 | 100 | 110 | 120 | 130 | 140 | 150 | 160 |
|---|---|---|---|---|---|---|---|---|
| Y value Natural skin | 30 | 34 | 37 | 42 | 51 | 56 | 58 | 56 |
| Example 1 | 32 | 35 | 38 | 44 | 48 | 53 | 55 | 53 |
| Comparative Example 1 | 33 | 35 | 38 | 43 | 47 | 52 | 50 | 49 |
| Comparative Example 2 | 33 | 37 | 39 | 45 | 48 | 51 | 51 | 48 |
| Comparative Example 3 | 33 | 36 | 38 | 44 | 47 | 47 | 46 | 40 |

### 2) Organoleptic Practical Use Test

The practical use test of the make-up cosmetic compositions of Examples 1 - 3 and Comparative Examples 1 - 5 was carried out using a practical use panel consisting of 50 testees. The evaluations were effected according to the following evaluation standard for each evaluation item.

### (Evaluation Standard)

| (Evaluation Score) Point | Conditions |
|---|---|
| 5 | Remarkably better when compared with Comparative Example 2 |
| 4 | Fairly better when compared with Comparative Example 2 |
| 3 | Similar to Comparative Example 2 |
| 2 | Little worse when compared with Comparative Example 2 |
| 1 | Heavily worse when compared with Comparative Example 2 |

| (Evaluation) | Average evaluation score |
|---|---|
| ⓞ | 4.0 - 5.0 |
| ○ | 3.0 - 3.9 |
| Δ | 2.0 - 2.9 |
| × | 1.0 - 1.9 |

**Table 4**

| Evaluation Item | Example | | Comparative Example | | | Example | Comparative Example | |
|---|---|---|---|---|---|---|---|---|
| | 1 | 2 | 1 | 2 | 3 | 3 | 4 | 5 |
| Presence of covering power | ⓞ | ⓞ | Δ | ○ | ⓞ | ○ | Δ | ⓞ |
| Extent of natural finish | ⓞ | ⓞ | ○ | ○ | Δ | ⓞ | ○ | Δ |
| Healthy and clear finish | ○ | ⓞ | ○ | ○ | Δ | ○ | ○ | Δ |
| Texture and noticeability of pore | ⓞ | ⓞ | Δ | ○ | Δ | ○ | ○ | Δ |
| Overall beautiful finish | ⓞ | ⓞ | Δ | ○ | Δ | ⓞ | ○ | Δ |
| Personal preference | ⓞ | ⓞ | ○ | ○ | Δ | ⓞ | ○ | Δ |

As shown in Table 4, the make-up cosmetic compositions according to the present invention can provide natural clear cosmetic finish, while covering defects on the skin. Furthermore, as shown in Example 2, when the titanated mica coated with the hollow spherical resin was used in combination with the other hollow spherical resin coated powder, the more healthy cosmetic finish closer to the natural skin was obtained.

### Example 4: Water in Oil (W/O) type emulsified liquid foundation

| | |
|---|---|
| Butylene glycol | 2.0 wt% |
| Silicone-treated mica | 10.0 |
| Silicone-treated iron oxide | 3.6 |
| Silicone-treated sericite | 7.0 |
| Barium sulfate coated with hollow spherical styrene-acryl copolymer resin (Preparation Example 3) | 13.0 |
| Volatile silicone | 31.2 |
| Squalane | 4.5 |
| Dimethylpolysiloxane (6 cs) | 3.0 |
| Sorbitan sesquiisostearate | 1.5 |
| Polyether modified silicone | 1.0 |
| Dispersing aid | 1.2 |
| Ion exchange water | 20.4 |
| Paraben | q.s. |
| Flavor | q.s. |
| Antioxidant | q.s. |

### Example 5: Oil-in-Water (O/W) emulsion type solid foundation

| | |
|---|---|
| Butylene glycol | 5.0 wt% |
| Organic titanate-treated sericite | 18.0 |
| Organic titanate-treated titanium dioxide fine powder | 6.0 |
| Organic titanate-treated iron oxide | 3.2 |
| Alumina coated with hollow spherical styrene-acryl copolymer resin (Preparation Example 4) | 7.0 |
| Titanated mica coated with hollow styrene-acryl copolymer resin (Preparation Example 2) | 5.0 |
| Dimethyl polysiloxane (6 cs) | 9.0 |
| Methylphenyl polysiloxane | 6.0 |
| Decamethylcyclopenta siloxane | 15.0 |
| Squalane | 2.0 |
| Tri(2-ethylhexanoic acid)glyceryl | 1.0 |
| Solid paraffin | 6.5 |
| Carnauba wax | 1.5 |
| Polyoxyethylene sorbitan fatty acid ester | 1.0 |
| Polyether-modified silicone | 1.5 |
| Ion exchange water | 12.2 |
| Paraben | q.s. |
| Flavor | q.s. |
| Antioxidant | q.s. |

### Example 6: Solid Powdery Foundation (Dual purpose)

| | |
|---|---|
| Silicone treated mica | 20.0 wt% |
| Silicone treated sericite | Balance |
| Silicone treated talc | 10.0 |
| Zinc oxide | 3.0 |
| Alumina coated with hollow spherical silicone resin (plate-like) | 15.0 |
| Silicone treated iron oxide | 4.0 |
| Antiseptics | q.s. |
| Methylphenyl polysiloxane (20 cs) | 4.0 |
| Squalane | 4.0 |
| Dimethyl polysiloxane (5000 cs) | 3.0 |
| Polyether-modified silicone | 1.5 |
| Sorbitan sesquiisostearate | 1.0 |
| Antioxidant | q.s. |
| Flavor | q.s. |

### Example 7: Solid Powdery Foundation (Cake)

| | |
|---|---|
| Silicone treated mica | 20.0 wt% |
| Silicone treated sericite | Balance |
| Talc | 10.0 |
| Kaolin | 15.0 |
| Alumina coated with hollow spherical polymethylmethacrylate resin (plate-like) | 12.0 |
| Silicone treated iron oxide | 3.8 |
| Antiseptic | q.s. |
| Methylphenyl polysiloxane | 4.0 |
| Dimethyl polysiloxane (10 cs) | 10.0 |
| Dimethylpolysiloxane (5000 cs) | 1.0 |
| Sorbitan sesquiisostearate | 1.5 |
| Hydrogenated castor oil | 1.5 |
| Antioxidant | q.s. |
| Flavor | q.s. |

As explained above, the make-up cosmetic composition using the composite powder according to the present invention can provide highly transparent, and natural finishing effects close to the natural skin, while covering defects on the skin. Such finishing is not known in the art.

## Claims

1. A composite powder comprising (a) a flake powder having (b) hollow spherical resin powder coated on the substantially entire surface thereof, the weight ratio of the powder (a): the powder (b) being 80:20 to 20:80.

2. A composite powder as claimed in claim 1, wherein the flake powder (a) is at least one powder selected from the group consisting of talc, mica, sericite, barium sulfate, alumina, boron nitride, synthetic mica, synthetic talc, zinc oxide, silica, titanated mica, lauroyl lysine, and iron oxide coated mica and having an average particle diameter of 1 to 80 µm.

3. A composite powder as claimed in claim 1, wherein the flake powder (a) is a combination of titanated mica and one or more other powders in the form of a flake.

4. A composite powder as claimed in claim 1, wherein the hollow spherical resin powder (b) is composed of at least one resin selected from the group consisting of acryl resins, nylon resins, styrene resins, silicone resins, and any copolymer resins thereof.

5. A composite powder as claimed in claim 1, wherein the hollow spherical resin powder (b) is composed of a styrene-acryl copolymer resin.

6. A composite powder as claimed in claim 1, wherein the hollow spherical resin powder (b) has an average hollow diameter of 0.1 - 0.5 µm and an average particle diameter of 0.3 - 1.5 µm.

7. A make-up cosmetic composition comprising a make-up base component and a composite powder according to claim 1.

8. A make-up cosmetic composition as claimed in claim 7, wherein the amount of the composite powder is 1.0 to 30.0% by weight, based upon the total weight of the cosmetic composition.
